(19) Europäisches Patentamt / European Patent Office / Office européen des brevets

(11) **EP 3 342 340 B1**

(12) **EUROPEAN PATENT SPECIFICATION**

(45) Date of publication and mention
of the grant of the patent:
**31.07.2019 Bulletin 2019/31**

(21) Application number: **17205872.9**

(22) Date of filing: **07.12.2017**

(51) Int Cl.:
*A61B 5/11* *(2006.01)*          *A61B 5/22* *(2006.01)*

(54) **SYSTEM FOR THE QUANTITATIVE EVALUATION OF THE INTERPERSONAL INTERACTION DURING THE LOCOMOTION AND OPERATION METHOD THEREOF**

SYSTEM ZUR QUANTITATIVEN BESTIMMUNG VON ZWISCHENMENSCHLICHE INTERAKTION WÄHREND DER FORTBEWEGUNG UND BETRIEBSVERFAHREN DAFÜR

SYSTÈME POUR L'ÉVALUATION QUANTITATIVE D'INTERACTIONS INTERPERSONNELLES PENDANT LA LOCOMATION ET PROCÉDÉ DE FONCTIONNEMENT DE CE DERNIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.12.2016 IT 201600132368**

(43) Date of publication of application:
**04.07.2018 Bulletin 2018/27**

(73) Proprietor: **Fondazione Santa Lucia**
**00179 Roma (IT)**

(72) Inventors:
• **SYLOS LABINI, Francesca**
**00179 Roma (IT)**
• **D'AVELLA, Andrea**
**00179 Roma (IT)**
• **CAPPELLINI, Germana**
**00179 Roma (IT)**
• **MARTINO, Giovanni**
**00179 Roma (IT)**
• **CATAVITELLO, Giovanna**
**00179 Roma (IT)**
• **LA SCALEIA, Valentina**
**00179 Roma (IT)**
• **LACQUAINITI, Francesco**
**00179 Roma (IT)**
• **IVANENKO, Yury**
**00179 Roma (IT)**

(74) Representative: **Tiburzi, Andrea et al**
**Barzanò & Zanardo Roma S.p.A.**
**Via Piemonte 26**
**00187 Roma (IT)**

(56) References cited:
**US-A- 4 674 330      US-A1- 2010 240 495**

• **DUCOURANT T ET AL: "Timing and distance characteristics of interpersonal coordination during locomotion", NEUROSCIENCE LETTERS, ELSEVIER, AMSTERDAM, NL, vol. 389, no. 1, 25 November 2005 (2005-11-25), pages 6-11, XP027653965, ISSN: 0304-3940 [retrieved on 2005-11-25]**
• **NESSLER J A ET AL: "Kinematic analysis of side-by-side stepping with intentional and unintentional synchronization", GAIT & POSTURE, ELSEVIER, AMSTERDAM, NL, vol. 31, no. 4, 1 April 2010 (2010-04-01), pages 527-529, XP027248544, ISSN: 0966-6362 [retrieved on 2010-02-23]**

**Description**

The present invention relates to a system for the quantitative evaluation of interpersonal interaction during locomotion.

The present invention also relates to an operation method of said system.

More specifically, the invention concerns a system of the aforementioned type, configured to measure a resulting force equal to the sum of the forces exerted on a detection device by at least two individuals during locomotion, to process this force and to provide a map showing the direction of this force in a reference system.

In particular, this resulting force is obtained by means of a contact or a haptic perception.

By contact or haptic perception it is intended a objects touch recognizing process, deriving from the combination between the tactile perception, given by the objects on the surface of the skin, and proprioception, which derives from the position of the hand with respect to the object.

In the following the description will be directed to a system capable of detecting a resulting force, processing this resulting force and providing a map, showing the direction of such a force in a reference system, relative to two individuals who, during the locomotion, exercise each a respective force on said detection device.

However, the same system should not be considered limited to this specific use.

Advantageously, such a system can be used for the study of any of motor coordination mechanism between two people, which requires the detection of forces exchanged between them.

As is well known, the ability to coordinate one's movements with those of others is one of the fundamental characteristics of different animal species and in particular of human species.

Since childhood, in fact, motor gestures must be able to respond and adapt to verbal, visual and tactile stimuli coming from other human beings.

The analysis of these responses and of the consequent motor adaptations can be used to study and monitor the operation of the human central nervous system at the level of complex sensorimotor integrations that regulate the mechanisms of inter-individual interaction, as mentioned in the Sebanz et al., 2006.

It is also possible to observe that two people who walk side by side tend to synchronize their pace, and it has been suggested by some studies such as Nessler and Gilliland, 2010 and Zivotofsky and Hausdorff, 2007, that this phenomenon could involve the neural networks activity of the "mirror neurons", which it is thought to play a role in the mechanisms of acquisition of new motor skills through the processes of imitation of the motor gestures.

On the basis of these hypotheses, it has been suggested, among other things, that this innate tendency to the synchronization could be exploited for the rehabilitation of the walk due to neurological damage, providing means to re-learn the appropriate motor patterns involved in locomotion (Nessler and Gilliland 2010; Sawers and Ting 2014).

The role and weight of the different ways through which this coordination can occur during the locomotion is still under study, as well as if the connection between two walking people can take place through a mechanical coupling and/or through an exchange of visual, auditory or tactile sensory information.

However, the few studies carried out so far suggest that, when two walking people are in contact through a mechanical and tactile coupling, they show the greatest degree of synchronization between their gaits compared to other ways of connection (Zivotofsky and Hausdorff, 2007).

Furthermore, systems have been developed for the analysis of the interaction of human forces with the environment in order to obtain a better understanding of the complex mechanisms that coordinate the movement and robots have been designed capable of emulating human skills in terms of movement and sensory response.

It is known a system for the study of the interaction between two people or for the bimanual control, comprising two portable aptic interfaces which, fixed on a plane, allow to perform bimanual or interactive tasks using a rigid or independent coupling.

This system, however, must be fixed on a plane and allows only the execution of the wrist movements, therefore it is not possible to evaluate the interaction strength during tasks that require the movement of the whole body, such as the locomotion.

Moreover, the movements of the wrist are bound to a single degree of freedom, the use of actuators introduces a number of errors due to mechanical frictions and delays and finally, the contact between subjects is not natural.

A haptic guide system is also known, wherein the robot adaptation mechanism is based on the measurement of the force applied by the subject in a specific direction.

The goal is to create an assistant capable of collaborating with a human subject while moving loads.

However, this system is cumbersome and the technology is not able to properly and naturally simulate the interaction developing with a human subject. DUCOURANT T ET AL: "Timing and distance characteristics of interpersonal coordination during locomotion", NEUROSCIENCE LETTERS, ELSEVIER, AMSTERDAM, NL, vol. 389, no. 1, November 2005, pages 6-11, ISSN: 0304-3940, the question of the interpersonal coordination when pairs of subjects walk simultaneously is addressed.

In light of the above, it is therefore an object of the present invention to provide a non-invasive system for the study of neurophysiological mechanisms that control the human motion.

A further object of the present invention is to provide a system for the quantitative evaluation of alterations of locomotor functions.

Another object of the present invention is to carry out the aforementioned analyses in real time.

A further object of the present invention is to provide the tools necessary for carrying out the method and the apparatuses, which perform this method.

It is therefore specific object of the present invention a system for the calculation of the forces exchanged between at least one first and one second individual during locomotion, said system comprising a forces detection device configured to detect the intensity of a first force exerted by said first individual on said forces detection device and the intensity of a second force exerted by said second individual on said forces detection device, a plurality of kinematic devices, each of which is configured to detect the position of said force detection device, and a plurality of motion data associated to said first individual and to said second individual, wherein said plurality of motion data comprises the position of said first individual, the position of said second individual and the position of said forces detection device, and a control logic unit, operatively connected to said forces detection device and to said plurality of kinematic devices, configured for acquiring the values of said first force and said second force from said forces detection device, calculating the intensity of the resultant force of said first force and said second force, acquiring the motion data from said plurality of kinematic devices, creating a map of the force distribution which shows the direction of said resultant force in a fixed reference system.

Further according to the invention, said forces detection device, comprises at least one first portion and one second portion, each portion being graspable from an individual, and a joining element for joining said first portion and said second portion, wherein said joining element comprises a sensor.

Still according to the invention, said sensor is a miniaturized three-dimensional sensor, such as a load cell or a piezoelectric sensor or a strain gauge, or the like.

Preferably according to the invention, said first portion and said second portion are made in a shape memory material, such as plastic material.

Further according to the invention, said plurality of kinematic devices comprises at least two infrared cameras, or three video cameras, or at least two inertial sensors.

Still according to the invention, each kinematic device of said plurality of kinematic devices is configured for detecting the position in the space of said at least two individuals during their locomotion side by side.

It is further object of the present invention a method for the calculation of the forces exchanged between at least one first and one second individual during locomotion, comprising the following steps:

acquiring the value of the intensity of a first force and the value of the intensity of a second force, each force being exerted from said at least one first individual and said second individual on a forces detection device;

calculating the intensity of the resultant force of said first force and said second force;

acquiring the motion data of said first individual and of said second individual during their locomotion;

synchronizing said motion data with at least one characteristic event of the gait cycle of each individual, such as the attack, the rest and the push;

calculating the three spatial components $F_x$, $F_y$, $F_z$, of the intensity of said resultant force with respect to a fixed reference system;

calculating the directional cosines of said resultant force;

providing a graphical representation of said directional cosines, as points lying on a sphere of unitary radius;

calculating the probabilistic distribution of said directional cosines on said sphere;

creating a map of the force distribution which shows the direction of said resultant force in said fixed reference system; and

displaying said map of the force distribution.

Preferably according to the invention, said method may comprise the following steps:

calculating the components $x_1$, $y_1$, $z_1$ of the approximate position of the center of mass of the first individual and the components $x_2$, $y_2$, $z_2$ of the approximate position of the center of mass of the second individual;

calculating the components 1, 1, 1 of the approximate velocity of the center of mass of the first individual and the components 2, 2, 2 of the approximate velocity of the center of mass of the second individual;

calculating the matrix of elasticity K and the matrix of damping D:

$$\begin{bmatrix} F_x \\ F_y \\ F_z \end{bmatrix} = K \begin{bmatrix} x_2 - x_1 \\ y_2 - y_1 \\ z_2 - z_1 \end{bmatrix} + D \begin{bmatrix} \dot{x}_2 - \dot{x}_1 \\ \dot{y}_2 - \dot{y}_1 \\ \dot{z}_2 - \dot{z}_1 \end{bmatrix}$$

The present invention will be now described, for illustrative but not limitative purposes, according to its preferred embodiments, with particular reference to the figures of the enclosed drawings, wherein:

figure 1 shows a schematic view of the system for the quantitative evaluation of the interpersonal interaction during the locomotion object of the present invention;

figure 2 shows a perspective top view of a first embodiment of a component of the system of figure 1;
figure 3 shows a perspective top view of a second embodiment of a component of the system of figure 1;
figure 4 shows a side perspective view of a third embodiment of a component of the system of figure 1;

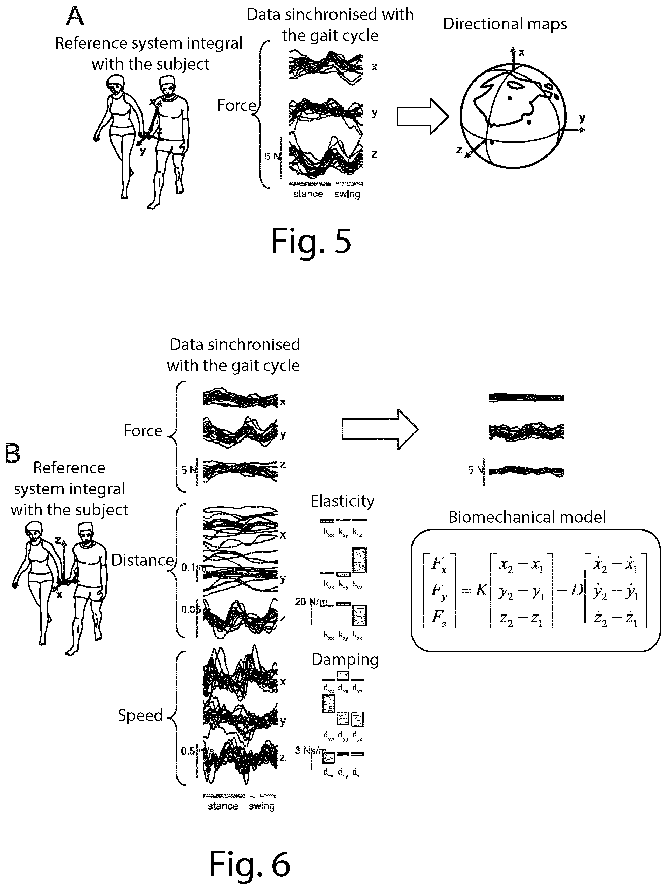

figure 5 shows a diagram concerning an operating mode of said system;
figure 6 shows a further diagram concerning an operating mode of said system; and

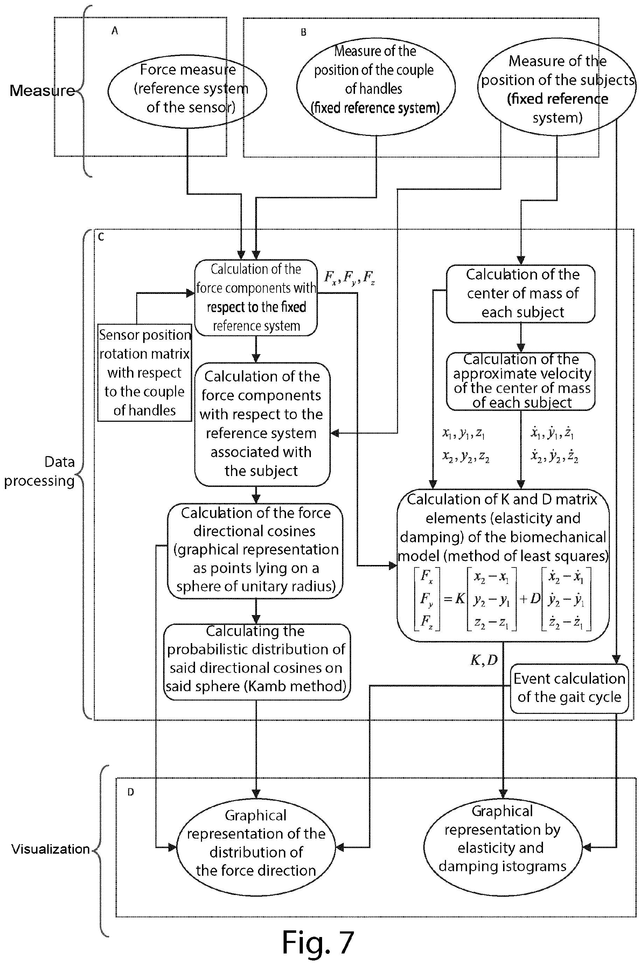

figure 7 shows a block diagram of the operation of the system object of the present invention.

In the various figures, similar parts will be indicated by the same reference numbers.

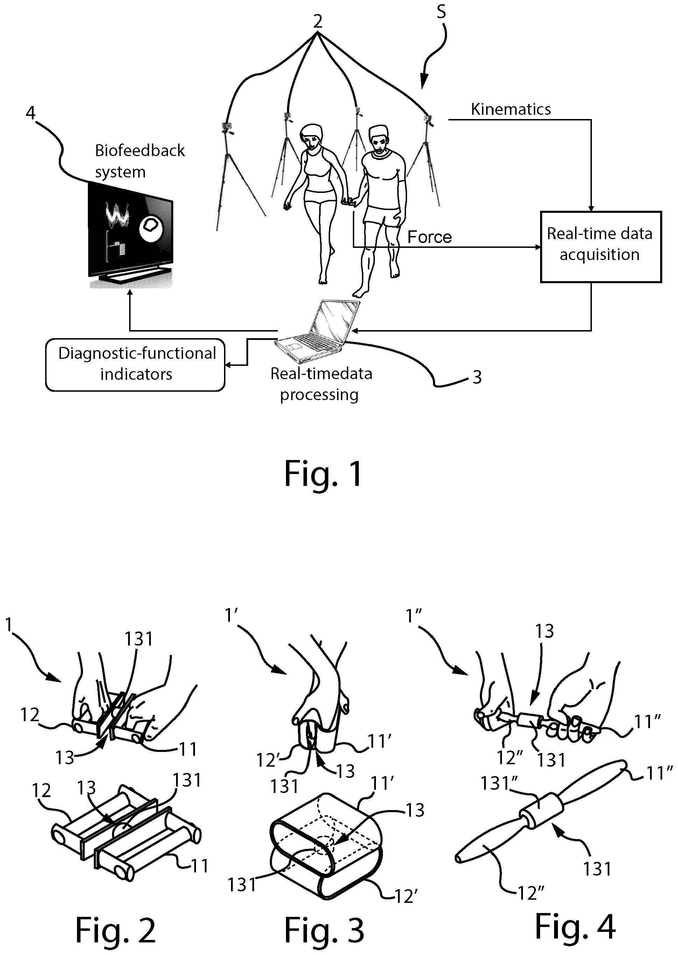

Referring to figure 1, a system S is described, configured to measure a force, process this force and provide a map showing the direction of such a force in a reference system.

In particular, this force is the resulting force given by the sum of the forces exerted on a detection device 1, 1', 1" (which is part of said system S) by at least two individuals during the locomotion.

Said system S comprises: a detection device 1, 1', 1" configured to detect the intensities of a first force exerted by a first individual and a second force exerted by a second individual on said detection device 1, 1', 1", a plurality of kinematic devices 2, each one of which configured to detect the position of said detecting device 1, 1', 1", as well as a plurality of motor data associated with said first individual and with said second individual, wherein said plurality of motor data comprises the position of said first individual and the position of said second individual, and a control logic unit 3, connected to said detection device 1, 1', 1" and to said plurality of kinematic devices 2, and configured for: acquiring the values of said first force and of said second force, synchronizing said data with at least one characteristic of the step cycle, calculating the intensity of the force resulting from said first force and said second force, acquiring the motor data from said plurality of kinematic devices 2, creating a map showing the direction of said resultant force in a reference system.

Furthermore, the system S can also include display means 4 for displaying the processing results.

With reference to figure 2, the detection device 1 allows the measurement of the interaction level between the two individuals, according to an internal reference system thereof.

In a first embodiment, which allows the use with the hands arranged in parallel, said detection device 1 comprises at least one first 11 and one second handle 12, which can be gripped by an individual.

The first 11 and second 12 handles are made of plastic material or any shape memory material.

The first 11 and second 12 handles are connected to each other by a connecting element 13 comprising a sensor 131.

Said sensor 131 is a miniaturized three-dimensional sensor, such as a load cell, a piezoelectric sensor, a strain gauge, and the like.

With reference to figure 3, the detection device 1', in a second embodiment, is shaped in such a way that, when used by two individuals, the palms of each hand are in contact with said detecting device and the fingers of the two hands are in contact with each other. It comprises at least ne first 11' and one second 12' handle, which can be gripped by an individual.

Said first 11' and second 12' handle are made of plastic material or any shape-memory material.

Said first 11' and second 12' handles are connected to each other by means of said connecting element 13, comprising said sensor 131.

With reference to figure 4, the the haptic contact detection device 1", in a third embodiment, which allows the use with the hands aligned, comprises at least a first 11" and a second 12" handle, which can be gripped by a individual.

Said first 11" and second 12" handle are made of plastic material, or any shape-memory material.

Said first 11" and second 12 " handles are connected to each other by means of said connecting element 13, which comprises said sensor 131.

Said detecting devices 1, 1' and 1" can be used separately or jointly in a same data collection session, as they allow the tactile contact of different surfaces of the hands of said at least two individuals.

Therefore, a joint use, allows to evaluate the way in which different coupling modalities influence interpersonal interaction in healthy subjects and in patients suffering from diseases that affect the locomotor system, whether they are elderly, adults or children.

Said detection device 1, 1', 1" is connected to said control logic unit 3 for sending the measured force data.

Said plurality of kinematic devices 2 comprises at least one first and one second infrared camera, or three video cameras, or at least two inertial sensors such as a gyroscope or optical sensors.

Said plurality of kinematic devices 2 detects a plurality of motor data, in a fixed reference system, integral with the laboratory, wherein the measurement takes place, where said plurality of motor data comprises the position of said at least two individuals in the space during the locomotion side by side, and in particular the synchronization of the kinematics of the different gaits of the two individuals, to measure in real time the three-dimensional position of a predetermined number of repere points positioned on the trunk, legs and arms of the two individuals.

Said plurality of kinematic devices 2 is operatively connected to said control logic unit 3 for sending the detected data of the movement of said at least two individuals.

Said control logic unit 3 is configured to process the intensities of said first force and of said second force as well as the motor data, previously described, as will be described in more detail below, and to create at the output a force distribution map showing the distribution of the resulting force in the time and preferably sound signals resulting from a calculation of the direction of the resulting force, of the intensity and of significant space-time parameters of the interaction of the two individuals during locomotion, while gripping said detection device 1, 1' and 1".

Said control logic unit 3 also includes calibration data relating to the geometrical conformation of each of said detecting devices 1, 1', 1", such as a rotation matrix of the position of said sensor 131 with respect to said first 11, 11', 11" and second 12, 12', 12" handle, which allow providing the data detected by said sensor 131 in its reference system.

Said control logic unit 3 sends said force distribution map to said display means 4, which may comprise a monitor or the like, to allow said at least two individuals to visualize the distribution of the force resulting during the locomotion.

The operation of the system S described above is as follows.

When it is desired studying the interaction level between at least two individuals walking side by side, said detection device 1, 1', 1" is provided, which the individuals hold by means of said first 11, 11', 11" and second 12, 12', 12" handle.

Said sensor 131 detects the intensity of the force exchanged between said at least two individuals during the locomotion, in a reference system of the sensor 131 itself, and sends said data to said control logic unit 3.

Said sensor 131, in fact, measures the forces that are applied to it, but, during the locomotion, it is not capable of detecting also its own position and therefore it can detect the forces only with respect to itself, therefore with respect to a reference system integral with itself.

Said plurality of kinematic devices 2 detects the position of said detecting device 1, 1', 1" in a fixed reference system, namely integral with the ground, and the position of said at least two individuals, in the same fixed reference system, and sends said data to said logic control unit 3.

Said logicl control unit 3 carries out the following calculations.

Initially it synchronizes the received data with at least one characteristic of the gait cycle, such as the start, the support and the thrust.

Subsequently it calculates the three spatial components $F_x$, $F_y$, $F_z$, of the intensity of the resulting force, with respect to the fixed reference system, taking into account said calibration data.

Then, it calculates the three spatial components of the intensity of the resulting force, relative to the fixed reference system.

Then, it calculates the directional cosines of the resulting force and provides a graphical representation of these cosines, as points lying on a sphere of unitary radius.

Subsequently, it calculates the probabilistic distribution of the directional cosines on the sphere by the known method of Kamb (Kamb, W. Barclay.) "Ice Petrofabric Observations from Blue Glacier, Washington, in Relation to Theory and Experiment." Journal of Geophysical Research 64, no. 11 (1959): 1891-1909). Next, it creates a map of the distribution of the force direction distribution.

At the same time, said control logic unit 3 performs an evaluation of the elasticity and the damping of the connection between said at least two individuals during the locomotion by the study of a biomechanical model, in which individuals are modeled as two material points whose three-dimensional positions in space correspond to the position of the respective center of mass, calculated by means of the motor data acquired with said plurality of kinematic devices 2.

In particular, said control logic unit 3 calculates the components $x_1$, $y_1$, $z_1$, of the approximate position of the center of mass of the first individual and the components $x_2$, $y_2$, $z_2$, of the position of the second individual.

Then it calculates the components $\dot{x}_1$, $\dot{y}_1$, $\dot{z}_1$ of the approximate velocity of the center of mass of the first individual and the components $\dot{x}_2$, $\dot{y}_2$, $\dot{z}_2$ of the approximate velocity of the center of mass of the second individual.

Subsequently it calculates the elasticity matrix K and the damping matrix D of said biomechanical model using the method of least squares:

$$\begin{bmatrix} F_x \\ F_y \\ F_z \end{bmatrix} = K \begin{bmatrix} x_2 - x_1 \\ y_2 - y_1 \\ z_2 - z_1 \end{bmatrix} + D \begin{bmatrix} \dot{x}_2 - \dot{x}_1 \\ \dot{y}_2 - \dot{y}_1 \\ \dot{z}_2 - \dot{z}_1 \end{bmatrix}$$

Finally, said control logic unit provides a graphical representation through histograms of the elasticity and the damping.

Said graphic representation of the force distribution and said histograms are displayed on said display device 4.

As it is apparent from the above description, said system S allows to calculate quantitatively and in real time the interaction between two individuals during the locomotion side by side.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. System (S) for the calculation of the forces exchanged between at least one first and one second individual during locomotion, said system comprising:

   a forces detection device (1, 1', 1") configured to detect the intensity of a first force exerted by said first individual on said forces detection device (1, 1', 1") and the intensity of a second force exerted by said second individual on said forces detection device (1, 1', 1"),
   a plurality of kinematic devices (2), each of which is configured to detect the position of said force detection device (1, 1', 1"), and a plurality of motion data associated to said first individual and to said second individual, wherein said plurality of motion data comprises the position of said first individual, the position of said second individual and the position of said forces detection device (1, 1', 1"), and
   a control logic unit (3), operatively connected to said forces detection device (1, 1', 1") and to said plurality of kinematic devices (2), configured for:

      acquiring the values of said first force and said second force from said forces detection device (1, 1', 1"),
      calculating the intensity of the resultant force of said first force and said second force,
      acquiring the motion data from said plurality of kinematic devices (2),
      creating a map of the force distribution which shows the direction of said resultant force in a fixed reference system.

2. System (S) according to the preceding claim **characterized in that** said forces detection device (1, 1', 1"), comprises
   at least one first portion (11, 11', 11") and one second portion (12, 12', 12"), each portion being graspable from an individual, and
   a joining element (13) for joining said first portion (11, 11', 11") and said second portion (12, 12', 12"), wherein said joining element (13) comprises a sensor (131).

3. System (S) according to the preceding claim **characterized in that** said sensor (131) is a miniaturized three-dimensional sensor, such as a load cell or a piezoelectric sensor or a strain gauge, or the like.

4. System (S) according to claim 2 or 3, **characterized in that** said first portion (11, 11', 11") and said second portion (12, 12', 12") are made in a shape memory material, such as plastic material.

5. System (S) according to any one of the preceding claims, **characterized in that** said plurality of kinematic devices (2) comprises at least two infrared cameras, or three video cameras, or at least two inertial sensors.

6. System (S) according to any one of the preceding claims, **characterized in that** each kinematic device (2) of said plurality of kinematic devices (2) is configured for detecting the position in the space of said at least two individuals during their locomotion side by side.

7. Method for the calculation of the forces exchanged between at least one first and one second individual during locomotion, comprising the following steps:

   acquiring the value of the intensity of a first force and the value of the intensity of a second force, each force being exerted from said at least one first individual and said second individual on a forces detection device (1, 1', 1");
   calculating the intensity of the resultant force of said first force and said second force;
   acquiring the motion data of said first individual and of said second individual during their locomotion;
   synchronizing said motion data with at least one characteristic event of the gait cycle of each individual, such as the attack, the rest and the push;
   calculating the three spatial components $F_x$, $F_y$, $F_z$, of the intensity of said resultant force with respect to a fixed reference system;
   calculating the directional cosines of said resultant force;
   providing a graphical representation of said directional cosines, as points lying on a sphere of unitary radius;
   calculating the probabilistic distribution of said directional cosines on said sphere;
   creating a map of the force distribution which shows the direction of said resultant force in said fixed reference system; and
   displaying said map of the force distribution.

8. Method according to the preceding claim **characterized in that** it further comprises the following steps:

   calculating the components $x_1$, $y_1$, $z_1$ of the approximate position of the center of mass of the first individual and the components $x_2$, $y_2$, $z_2$ of the approximate position of the center of mass of the second individual;
   calculating the components 1, 1, 1 of the approximate velocity of the center of mass of the first individual and the components 2, 2, 2 of the approximate velocity of the center of mass of the second individual;
calculating the matrix of elasticity K and the matrix of damping D:

$$\begin{bmatrix} F_x \\ F_y \\ F_z \end{bmatrix} = K \begin{bmatrix} x_2 - x_1 \\ y_2 - y_1 \\ z_2 - z_1 \end{bmatrix} + D \begin{bmatrix} \dot{x}_2 - \dot{x}_1 \\ \dot{y}_2 - \dot{y}_1 \\ \dot{z}_2 - \dot{z}_1 \end{bmatrix}$$

**Patentansprüche**

1. System (S) zur Berechnung der Kräfte, die zwischen mindestens einer ersten und einer zweiten Person während der Fortbewegung ausgetauscht werden, wobei das System umfasst:

   eine Krafterfassungsvorrichtung (1, 1', 1 "), die konfiguriert ist, um die Intensität einer ersten Kraft, die von der ersten Person auf die Krafterfassungsvorrichtung (1, 1', 1") ausgeübt wird, und die Intensität einer zweiten Kraft zu erfassen, die von der zweiten Person auf die Krafterfassungsvorrichtung (1, 1', 1") ausgeübt wird, eine Vielzahl von kinematischen Vorrichtungen (2), von denen jede konfiguriert ist, um die Position der Krafterfassungsvorrichtung (1, 1', 1") zu erfassen, und eine Vielzahl von Bewegungsdaten, die der ersten Person und der zweiten Person zugeordnet sind, wobei die Vielzahl von Bewegungsdaten die Position der ersten Person, die Position der zweiten Person und die Position der Krafterfassungsvorrichtung (1, 1', 1") umfasst, und
   eine Steuerlogikeinheit (3), die funktionsfähig mit der Krafterfassungsvorrichtung (1, 1', 1") und mit der Vielzahl von kinematischen Vorrichtungen (2) verbunden ist, konfiguriert für:

      Erfassen der Werte der ersten Kraft und der zweiten Kraft von der Krafterfassungsvorrichtung (1, 1', 1"),
      Berechnen der Intensität der resultierenden Kraft der ersten Kraft und der zweiten Kraft,
      Erfassen der Bewegungsdaten von der Vielzahl der kinematischen Vorrichtungen (2),
      Erzeugen einer Karte der Kraftverteilung, die die Richtung der resultierenden Kraft in einem festen Bezugssystem anzeigt.

2. System (S) nach dem vorstehenden Anspruch, **dadurch gekennzeichnet, dass** die Krafterfassungsvorrichtung (1, 1', 1") umfasst mindestens einen ersten Abschnitt (11, 11', 11") und einen zweiten Abschnitt (12, 12', 12"), wobei jeder Abschnitt von einer Person greifbar ist, und ein Verbindungselement (13) zum Verbinden des ersten Abschnitts (11, 11', 11") und des zweiten Abschnitts (12, 12', 12"), wobei das Verbindungselement (13) einen Sensor (131) umfasst.

3. System (S) nach dem vorstehenden Anspruch, **dadurch gekennzeichnet, dass** der Sensor (131) ein miniaturisierter dreidimensionaler Sensor ist, wie eine Wägezelle oder ein piezoelektrischer Sensor oder ein Dehnungsmessstreifen oder dergleichen.

4. System (S) nach Anspruch 2 oder 3, **dadurch gekennzeichnet, dass** der erste Abschnitt (11, 11', 11") und der zweite Abschnitt (12, 12', 12") aus einem Formgedächtnismaterial, wie Kunststoffmaterial, hergestellt sind.

5. System (S) nach einem der vorstehenden Ansprüche, **dadurch gekennzeichnet, dass** die Vielzahl von kinematischen Vorrichtungen (2) mindestens zwei Infrarotkameras oder drei Videokameras oder mindestens zwei Trägheitssensoren umfasst.

6. System (S) nach einem der vorstehenden Ansprüche, **dadurch gekennzeichnet, dass** jede kinematische Vorrichtung (2) der Vielzahl von kinematischen Vorrichtungen (2) konfiguriert ist, um die Position im Raum der mindestens zwei Personen während ihrer Fortbewegung nebeneinander zu erfassen.

7. Verfahren zum Berechnen der Kräfte, die zwischen mindestens einer ersten und einer zweiten Person während der Fortbewegung ausgetauscht werden, umfassend die folgenden Schritte:

   Erfassen des Wertes der Intensität einer ersten Kraft und des Wertes der Intensität einer zweiten Kraft, wobei jede Kraft von der mindestens einen ersten Person und der zweiten Person auf eine Krafterfassungsvorrichtung (1, 1', 1") ausgeübt wird;
   Berechnen der Intensität der resultierenden Kraft der ersten Kraft und der zweiten Kraft;
   Erfassen der Bewegungsdaten der ersten Person und der zweiten Person während ihrer Fortbewegung;
   Synchronisieren der Bewegungsdaten mit mindestens einem charakteristischen Ereignis des Gangzyklus jeder Person, wie dem Vorstoß, der Ruhe und dem Vorantreiben;
   Berechnen der drei räumlichen Komponenten $F_x$, $F_y$, $F_z$, der Intensität der resultierenden Kraft in Bezug auf ein festes Bezugssystem;
   Berechnen der Richtungscosinus der resultierenden Kraft;

Bereitstellen einer grafischen Darstellung der Richtungskosinus als Punkte, die auf einer Kugel mit einheitlichem Radius liegen;
Berechnen der probabilistischen Verteilung der Richtungskosinus auf der Kugel;
Erzeugen einer Karte der Kraftverteilung, die die Richtung der resultierenden Kraft in dem festen Bezugssystem anzeigt; und
Anzeigen der Karte der Kraftverteilung.

8. Verfahren nach dem vorstehenden Anspruch, **dadurch gekennzeichnet, dass** es weiter die folgenden Schritte umfasst:

Berechnen der Komponenten $x_1$, $y_1$, $z_1$ der ungefähren Position des Massenmittelpunkts der ersten Person und der Komponenten $x_2$, $y_2$, $z_2$ der ungefähren Position des Massenmittelpunkts der zweiten Person;
Berechnen der Komponenten 1, 1, 1 der ungefähren Geschwindigkeit des Massenmittelpunktes der ersten Person und der Komponenten 2, 2, 2 der ungefähren Geschwindigkeit des Massenmittelpunktes der zweiten Person;
Berechnen der Matrix der Elastizität K und der Matrix der Dämpfung D:

$$\begin{bmatrix} F_x \\ F_y \\ F_z \end{bmatrix} = K \begin{bmatrix} x_2 - x_1 \\ y_2 - y_1 \\ z_2 - z_1 \end{bmatrix} + D \begin{bmatrix} \dot{x}_2 - \dot{x}_1 \\ \dot{y}_2 - \dot{y}_1 \\ \dot{z}_2 - \dot{z}_1 \end{bmatrix}$$

**Revendications**

1. Système (S) pour le calcul des forces échangées entre au moins un premier et un second individu pendant un déplacement, ledit système comprenant :

un dispositif de détection de forces (1, 1', 1") configuré pour détecter l'intensité d'une première force exercée par ledit premier individu sur ledit dispositif de détection de forces (1, 1', 1") et l'intensité d'une seconde force exercée par ledit second individu sur ledit dispositif de détection de forces (1, 1', 1"),
une pluralité de dispositifs cinématiques (2), chacun étant configuré pour détecter la position dudit dispositif de détection de forces (1, 1', 1") et une pluralité de données de mouvement associées audit premier individu et audit second individu, dans lequel ladite pluralité de données de mouvement comprend la position dudit premier individu, la position dudit second individu et la position dudit dispositif de détection de forces (1, 1', 1"), et
une unité logique de commande (3), reliée de manière opérationnelle audit dispositif de détection de forces (1, 1', 1") et à ladite pluralité de dispositifs cinématiques (2), configurée pour :

acquérir les valeurs de ladite première force et de ladite seconde force auprès dudit dispositif de détection de forces (1, 1', 1"),
calculer l'intensité de la force résultante de ladite première force et de ladite seconde force,
acquérir les données de mouvement auprès de ladite pluralité de dispositifs cinématiques (2),
créer une carte de la répartition de forces qui montre la direction de ladite force résultante dans un système de référence fixe.

2. Système (S) selon la revendication précédente **caractérisé en ce que** ledit dispositif de détection de forces (1, 1', 1"), comprend
au moins une première partie (11, 11', 11") et une seconde partie (12, 12', 12"), chaque partie pouvant être saisie à partir d'un individu, et
un élément de liaison (13) pour relier ladite première partie (11, 11', 11") et ladite seconde partie (12, 12', 12"), dans lequel ledit élément de liaison (13) comprend un capteur (131).

3. Système (S) selon la revendication précédente **caractérisé en ce que** ledit capteur (131) est un capteur tridimensionnel miniaturisé, tel qu'un capteur de force ou un capteur piézoélectrique ou une jauge de contrainte ou similaire.

4. Système (S) selon la revendication 2 ou 3, **caractérisé en ce que** ladite première partie (11, 11', 11") et ladite seconde partie (12, 12', 12") sont réalisées en un matériau à mémoire de forme, tel qu'un matériau plastique.

5. Système (S) selon l'une quelconque des revendications précédentes, **caractérisé en ce que** ladite pluralité de dispositifs cinématiques (2) comprend au moins deux caméras infrarouges ou trois caméras vidéo ou au moins deux capteurs d'inertie.

6. Système (S) selon l'une quelconque des revendications précédentes, **caractérisé en ce que** chaque dispositif cinématique (2) de ladite pluralité de dispositifs cinématiques (2) est configuré pour détecter la position dans l'espace desdits au moins deux individus pendant leur déplacement côte à côte.

7. Méthode de calcul des forces échangées entre au moins un premier et un second individu pendant un déplacement, comprenant les étapes suivantes :

l'acquisition de la valeur de l'intensité d'une première force et de la valeur de l'intensité d'une seconde force, chaque force étant exercée depuis ledit au moins un premier individu et ledit second individu sur un dispositif de détection de forces (1, 1', 1"),

le calcul de l'intensité de la force résultante de ladite première force et de ladite seconde force ;

l'acquisition des données de mouvement dudit premier individu et dudit second individu pendant leur déplacement ;

la synchronisation desdites données de mouvement avec au moins un événement caractéristique du cycle de marche de chaque individu, tel que l'attaque, le repos et la poussée ;

le calcul des trois composantes spatiales $F_x$, $F_y$, $F_z$ de l'intensité de ladite force résultante par rapport à un système de référence fixe ;

le calcul des cosinus directionnels de ladite force résultante ;

la fourniture d'une représentation graphique desdits cosinus directionnels, en tant que points situés sur une sphère de rayon unitaire ;

le calcul de la distribution probabiliste desdits cosinus directionnels sur ladite sphère ;

la création d'une carte de répartition de forces qui montre la direction de ladite force résultante dans ledit système de référence fixe ; et

l'affichage de ladite carte de la distribution de forces.

8. Procédé selon la revendication précédente **caractérisé en ce qu'**il comprend en outre les étapes suivantes :

le calcul des composantes $x_1$, $y_1$, $z_1$ de la position approximative du centre de gravité du premier individu et des composantes $x_2$, $y_2$, $z_2$ de la position approximative du centre de gravité du second individu ;

le calcul des composantes 1, 1, 1 de la vitesse approximative du centre de gravité du premier individu et des composantes 2, 2, 2 de la vitesse approximative du centre de gravité du second individu ;

le calcul de la matrice d'élasticité K et de la matrice d'amortissement D :

$$\begin{bmatrix} F_x \\ F_y \\ F_z \end{bmatrix} = K \begin{bmatrix} x_2 - x_1 \\ y_2 - y_1 \\ z_2 - z_1 \end{bmatrix} + D \begin{bmatrix} \dot{x}_2 - \dot{x}_1 \\ \dot{y}_2 - \dot{y}_1 \\ \dot{z}_2 - \dot{z}_1 \end{bmatrix}$$

Fig. 1

Fig. 2

Fig. 3

Fig. 4

Fig. 5

Fig. 6

Measure

A
Force measure
(reference system
of the sensor)

B
Measure of the
position of the couple
of handles
(fixed reference system)

Measure of the
position of the subjects
(fixed reference
system)

C

Calculation of the
force components with
respect to the fixed
reference system

$F_x, F_y, F_z$

Sensor position
rotation matrix
with respect
to the couple
of handles

Calculation of the
force components
with respect to the
reference system
associated with
the subject

Data
processing

Calculation of the force
directional cosines
(graphical representation
as points lying on a
sphere of unitary radius)

Calculating the
probabilistic distribution of
said directional cosines on
said sphere (Kamb method)

Calculation of the
center of mass of
each subject

Calculation of the
approximate velocity
of the center of mass
of each subject

$x_1, y_1, z_1$  $\dot{x}_1, \dot{y}_1, \dot{z}_1$
$x_2, y_2, z_2$  $\dot{x}_2, \dot{y}_2, \dot{z}_2$

Calculation of K and D matrix
elements (elasticity and
damping) of the biomechanical
model (method of least squares)

$$\begin{bmatrix} F_x \\ F_y \\ F_z \end{bmatrix} = K \begin{bmatrix} x_2 - x_1 \\ y_2 - y_1 \\ z_2 - z_1 \end{bmatrix} + D \begin{bmatrix} \dot{x}_2 - \dot{x}_1 \\ \dot{y}_2 - \dot{y}_1 \\ \dot{z}_2 - \dot{z}_1 \end{bmatrix}$$

$K, D$

Event calculation
of the gait cycle

D

Graphical
representation of the
distribution of
the force direction

Graphical
representation by
elasticity and
damping istograms

Visualization

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Timing and distance characteristics of interpersonal coordination during locomotion. **DUCOURANT T et al.** NEUROSCIENCE LETTERS. ELSEVIER, November 2005, vol. 389, 6-11

- **KAMB, W. BARCLAY.** Ice Petrofabric Observations from Blue Glacier, Washington, in Relation to Theory and Experiment. *Journal of Geophysical Research,* 1959, vol. 64 (11), 1891-1909